# EUROPEAN PATENT SPECIFICATION

(11) **EP 2 080 493 B1**
(45) Date of publication and mention of the grant of the patent: **14.03.2012**
(21) Application number: 08167210.7
(22) Date of filing: 22.10.2008
(51) Int. Cl.: A61F 5/44

(54) **Two chamber drainable ostomy pouch**
Entwässerbarer Ostomiebeutel mit zwei Kammern
Poche de stomie vidable à deux chambres

(30) Priority: 21.01.2008 US 22477 P; 21.01.2008 EP 08100675
(43) Date of publication of application: 22.07.2009
(73) Proprietor: Hollister Incorporated, Libertyville IL 60048 (US)
(72) Inventor: Winther, Knud, 3070 Snekkersten (DK)
(74) Representative: Høiberg A/S

(56) References cited:
- WO-A-03/086250
- GB-A- 2 413 083
- US-A- 5 690 623

## Description

The present disclosure relates to a drainable ostomy pouch comprising:
- a first or proximal and a second or distal side wall of flexible sheet material sealed to each other for defining a cavity therebetween for receiving human stomal discharge through an aperture in said proximal side wall and an elongate drainage portion extending downwards in a longitudinal direction of the pouch, said drainage portion ending in a drainage outlet extending transversely to the longitudinal direction and formed along the bottom edges of the first and second side walls,
- a first or proximal and a second or distal flexible stiffening strip located near the drainage outlet and extending transversely to the longitudinal direction and being attached, for instance by heat sealing or adhesion, to the outer surfaces of the first and second side walls, respectively, of the drainage portion,
- a third, intermediate wall of flexible sheet material arranged between the proximal and distal side walls for dividing the cavity into two compartments, a proximal faeces compartment and a distal flatus gas compartment, and ending at a transversely extending bottom edge located in the vicinity of the drainage outlet, and
- securing means for securing a coil comprising the first and second stiffening strips and the first, second and third walls, the coil being formed by rotating the strips upwards at least 360 degrees, preferably at least 540 degrees,

Such ostomy pouches should be easy to drain without risking soiling of clothes, and they should be easy to close securely after being drained. Furthermore, it is essential that they should be amenable to being cleaned after drainage as any stomal effluent left in the region of the drainage outlet when the pouch is closed after being drained will give rise to unpleasant odours.

A drainable ostomy pouch of the type indicated above is disclosed in PCT patent application WO 03/086250 where the bottom edge of the intermediate wall located between the two stiffening strips and is free to move relative to the distal and proximal walls. When cleaning the drainage outlet after draining the pouch through the drainage outlet, the stiffening strips are bowed or arcuately flexed outwardly away from each other to open the drainage outlet and the interior of the pouch near the drainage outlet is cleaned with a cleaning implement such as, for example, a tissue, a cotton ball, or a cotton swab. However, the bottom edge of the intermediate wall interferes with the cleaning of the interior of the drainage outlet because it is loose and therefore prevents a cleaning implement such as a tissue in contacting all the soiled interior surfaces near the drainage outlet. Further, because it is loose, the bottom edge of the intermediate wall may tend to flex in the direction of the first or proximal wall, possibly clinging to that wall by reason of the faecal matter therebetween, thereby obstructing the discharge of such matter and interfering with the cleaning of the drainage outlet.

One of the objects of the present invention is to provide a drainable ostomy pouch of the type indicated where the cleaning of the drainage outlet and the interior region near the drainage outlet is facilitated.

This object may be achieved by the intermediate wall being attached at one or more spot welds or adhesive spots to the distal wall in the vicinity of the bottom edge of the intermediate wall such that if the stiffening strips are arcuately flexed away from each other to open the drainage outlet the intermediate wall will follow the distal wall and not interfere with the cleaning of the drainage outlet after the pouch is emptied through the drainage outlet.

Hereby, the bottom edge region of the intermediate wall will be fixated relative to the distal wall, and the bottom portion of the proximal faeces compartment adjacent the drainage outlet may be thoroughly cleaned without interference from the bottom edge of the intermediate wall.

In the currently preferred embodiment the bottom edges of the first and second walls and the bottom edge of the intermediate wall generally coincide. This simplifies the production of the pouch. Although the intermediate wall may be attached to the distal wall by adhesive it is currently preferred that the intermediate wall is attached to the distal wall by means of welding.

The welding is in the form of one or more spot welds, preferably four mutually spaced spot welds such that preferably two or more channels for draining any stomal effluent in the flatus gas compartment and for the escape of flatus gas that may be retained therein are formed between the spot welds.

Advantageously, the stiffening strips may be generally rectangular and they may generally overlie each other in generally parallel side-by-side relation. Hereby, the flexing of the strips away from each other for opening the drainage outlet for drainage and cleaning is facilitated.

Preferably, the bottom edges of the stiffening strips are located at a distance from the drainage outlet that is less than 10mm, preferably less than 5mm, most preferably less than 2mm. Hereby, the amount of flexible film forming the bottom portion of the three walls not supported by the stiffening strips is kept at a minimum as it is much more difficult to clean the region of the discharge opening if the films are not supported by the stiffening strips.

Preferably, the intermediate wall is attached to the distal wall at a distance from the drainage outlet that is less than 15mm, preferably less than 10mm, most preferably less than 8mm. Hereby the amount of loose flexible film of the intermediate wall not fixated to the distal wall is kept at a minimum which is advantageous for the cleaning process.

Advantageously, one or more flatus gas venting apertures, preferably a plurality of such apertures, may be provided in the intermediate wall and a flatus gas venting aperture may be provided in the distal wall and the first venting apertures may have a maximum dimension of between 0.01 and 0.5mm.

In the following, the invention, together with further objects and advantages, will best be understood by reference to the currently preferred embodiment thereof shown, solely be way of example, in the accompanying drawings, where:
Figs. 1 and 2 are schematic plan views of the proximal and distal sides, respectively, of the currently preferred embodiment of a pouch according to the disclosure,
Fig. 3 is a schematic enlarged scale cross-sectional view of the elongate drainage portion of the pouch of Figs. 1-2 taken along line A-A in Fig. 1, and
Fig. 4 is a schematic enlarged scale cross-sectional view taken along line B-B in Fig. 3 with the stiffening strips flexed away from each other to open the drainage outlet.

The ostomy pouch according to the disclosure may be part of a one- piece or of a two-piece ostomy appliance.

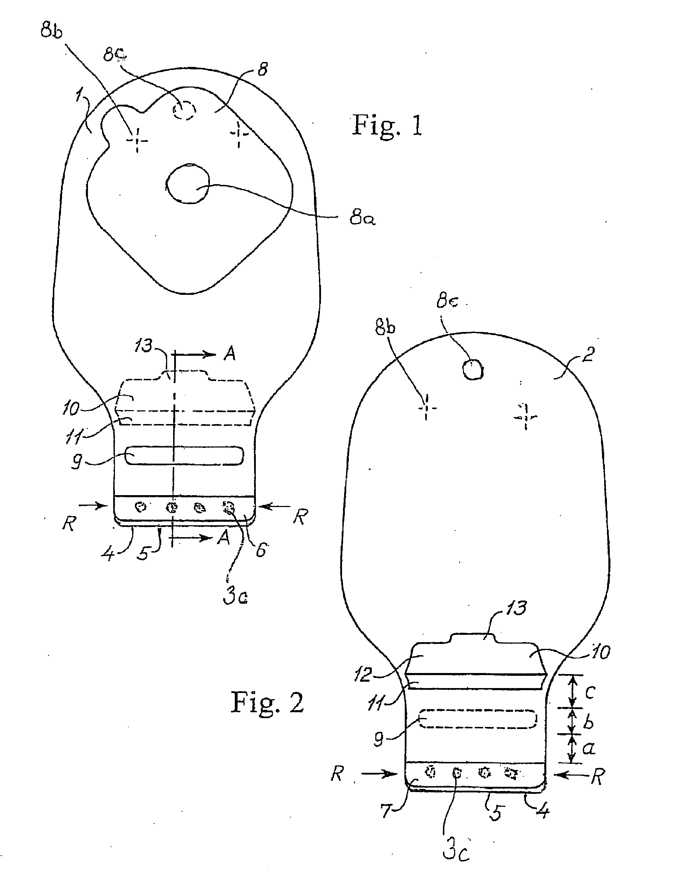

Referring now to Figs 1-3, the currently preferred embodiment of an ostomy pouch according to the invention comprises three sheets or films of a liquid and gas impermeable flexible material, a body side or proximal film 1, an opposed or distal film 2 and an intermediate film 3 having a bottom edge 4 and dividing the pouch into a flatus gas chamber 3a and a faeces chamber 3b.

An elongate drainage portion extends downwards from the main portion of the pouch and terminates in a drainage outlet 5 that extends along the bottom edges of the three films 1-3 and more specifically, is defined by and located between the bottom edges of films 1 and 3.

A proximal stiffening strip 6 and a distal stiffening strip 7 of a relatively stiff, flexible plastic material such as PET, nylon, HDPE or LDPE are attached to the outer surfaces of the lower ends of the films 1 and 2, respectively, and extend adjacent to and along the entire length of the drainage outlet 5.

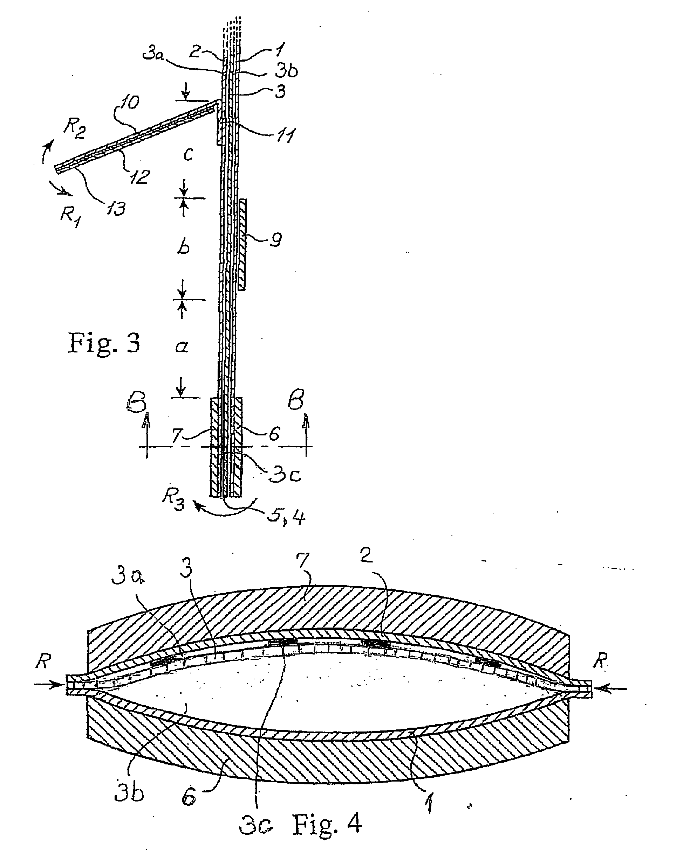

The bottom edge 4 of the intermediate film 3 coincides with the bottom edges of films 1 and 2. A row of four mutually spaced spot welds 3c spaced from the bottom edge 4 attach the lower end of the intermediate film 3 to the lower end of the distal film 2 such that the lower end of the intermediate film 3 is forced to follow the lower end of the distal film 2 when the stiffening strips 6 and 7 are flexed away from each other as illustrated in Fig.4.

A face plate 8 of a skin friendly adhesive barrier material is attached to film 1. In the case of a one-piece appliance (as shown), the face plate 8 is permanently attached to the film 1. However, if desired, the face plate and film 1 may be selectively detachable (not shown), in which case the appliance would be of a so-called two-piece construction. The face plate 8 serves to attach the pouch to the peristomal skin surface of a wearer of the ostomy pouch in a manner well known in the art.

The face plate 8 and the film 1 to which the face plate is attached are provided with aligned stoma-receiving apertures 8a or, alternatively, with aligned starter openings which may be enlarged at the time of application by cutting with scissors, all as well known in the art. The apertures 8a allow stoma effluents such as faeces and flatus gas to enter the pouch cavity.

The intermediate film 3 is provided near the upper end thereof with two pairs of cross cuts defining two openings 8b for allowing flatus gasses to pass from the faeces chamber 3b to the flatus gas chamber 3a. The flatus gasses then exit from the flatus gas chamber 3a through a flatus gas venting opening 8c in the distal wall 2. The intermediate film 3 prevents or restrains passage of faecal material from chamber 3b to chamber 3a so as not to clog a not shown flatus gas filter arranged so as to cover the a flatus gas venting opening 8c, as shown and described in US patent 5,690,623.

A strip 9 of an interlocking means such as Velcro (hook or loop portion), DUOTEC from G. Binder GmbH & Co., Germany (interlocking mushroom elements) or well known repeatedly releasable adhesive coatings, is attached to film 1.

A flap 10 of flexible film material may be attached to film 1 by welding or adhering an edge portion 11 of flap 10 to film 2. A strip 12 of a corresponding interlocking means such as Velcro (loop or hook portion), DUOTEC or the repeatedly releasable adhesive coating is attached to the flap 10.

The flap 10 is shown in different positions in Figs. 1, 2 and in Fig. 3, the position of flap 10 shown in Fig. 3 being rotated in the direction R1 compared to the position of flap 10 shown in Figs.1 and 2 where the flap 10 abuts the film 2, i.e. being rotated as far as possible in the direction R2. The flap 10 may include a gripping tab 13 for being gripped by the user's fingers.

In use, the faeces chamber 3b of the pouch with the discharge aperture 5 closed as described below is gradually filled with faeces while the flatus gasses are vented through the venting openings 8b and the flatus gas opening 8c. It may happen that some liquid faeces enters the flatus gas chamber 3a through the openings 8b, especially if the ostomate is physically active or during the night where pressure can be exerted on the pouch pressing liquid faeces through the openings 8b.

When it is necessary to empty the pouch, it is brought into the open configuration shown in Figs. 1-4 where the drainage outlet 5 is open. The faeces in the faeces chamber 3b are pressed out through the opening 5 and any faeces in the flatus gas chamber 3a are pressed out through the spaces or channels between the spot welds 3c.

The discharge portion of the pouch is closed in the following manner after the chambers 3a and 3b have been emptied through the drainage outlet 5 as described above:

The strips 6 and 7 are pressed together to squeeze any remaining matter out from the area between the strips 6 and 7. Then the strips are pulled or flexed apart by applying finger pressure to the ends thereof in the direction of arrows R in Figs. 1 and 2 and 4 so as to be able to access the faeces between the strips 6 and 7 at the drainage outlet 5 for removal thereof. The bottom edge portions of the films 1-3 along the drainage outlet 5 are then wiped clean with a cleaning implement, such as, for example, a tissue.

Because of the attachment of the intermediate film 3 to the distal film 2, the bottom end portion of film 3 does not make it difficult to clean the drainage outlet 5 by being loose and thereby interfering with the action of the cleaning implement.

This is illustrated in Fig. 4 (where the thickness of the strips 6 and 7 and the films 1-3 is exaggerated for the sake of clarity) showing that the drainage outlet 5 is maintained open by flexing the strips 6 and 7 apart by finger pressure applied to the ends thereof in the directions represented by the arrows R. Thereby, the interior of the bottom part of the drainage portion is easily accessible for being cleaned, for instance with a cleaning tissue, without interference from the bottom edge portion of the intermediate film 3 as this edge portion is held close to the bottom edge portion of distal film 2 by the spot welds 3c.

Thereafter the strips 6 and 7 are rotated clock-wise in the direction of arrow R3 in Fig. 3 until strip 7 abuts the area a of film 2. Thereafter the strips 6 and 7 are rotated 15 once more clock-wise until strip 6 abuts area b of film 2. Finally, the strips 6 and 7 are rotated one last time clock-wise such that the coil formed by the strips 6, 7 and 9 as well as the portions of films 1, 2 and 3 involved abuts the area c of film 2 with the strip 9 of Velcro or the like located such that the corresponding strip 12 of Velcro or the like can engage it by rotating the flap 10 in the direction R1 until the two Velcro strips 9 and 12 abut and engage each other for locking the coil in the final closed and secured position of the discharge portion.

In this closed coiled position the upper edge of the coil should preferably fit snugly and tightly in the angle formed between the portion 11 and the rest of the flap 10 such that pressure is exerted thereby on the coil and the portion 11 which is beneficial to the stability of the closure. The stiffness of the strips 6 and 7 also is helpful to achieve the stability of the closure.

The angle or fold between the portion 11 and the rest of the flap 10 furthermore affords the advantage that the flap 10 in its relaxed state will tend to be in an open position not abutting film 2 in which it is easy to grip when rolling the discharge portion up.

When the pouch is to be drained, the flap 10 is gripped by the tab 13 and pulled such that the strips 9 and 12 are disengaged so that the coil can be unrolled by rotating the strips 6 and 7 three times in the counter clock-wise direction, as oriented in Fig. 3.

The strips 6 and 7 may be used to control the rate of drainage of the pouch by opening and closing the drainage outlet 5 by pressing more or less forcefully on the ends of the strips 6 and 7 in the direction of the arrows R so that they flex away from and towards each other, respectively.

By locating the edge 4 as shown it is ensured that film 3 is held flat without wrinkles and folded just as many times as the films 1 and 2 such that there is no risk of leaks below the intermediate film 3 between the flatus gas chamber 3a and the faeces chamber 3b in the rolled-up, closed configuration of the drainage portion.

Although four mutually spaced spot welds 3c are shown, more or less the same effect will be achieved with a single centrally located spot weld or two, three or five mutually spaced spot welds. The shown four spot welds 3c are currently preferred because it gives the best visual impression. The spot welds 3c may be substituted by adhesive spots.

The two openings 8b in the intermediate film wall may be substituted by a number of small perforations with a size of for instance 0.01mm to 0.5mm. In such case, it will not be possible for appreciable amounts of liquid effluent to enter the flatus chamber 3b and therefore the spot welds or adhesive drops may be substituted by a continuous weld or continuous strip of adhesive extending across the entire width of the discharge portion as there then is no need for channels to allow effluent to be emptied out of the flatus gas chamber 3b.

The bottom edge 4 of the intermediate wall 3 may be located higher than the bottom edges of the films 1 and 2. However, the bottom edge should be in the vicinity of the drainage opening, i.e. not higher than the top edges of the stiffening strips such that the intermediate film is folded at least twice when the strips 6 and 7 are rotated upwards at least 360 degrees, preferably 540 degrees.

Although the stiffening strips 6 and 7 are shown overlying each other, the strips may be offset in a direction extending transversely to the longitudinal direction of the drainage portion so that each strip has one end portion extending beyond the corresponding end portion of the other of the offset strips as disclosed in US patent No. 6,887,222 and PCT patent application WO 03/086250.

## Claims

1. A drainable ostomy pouch comprising:
- a first or proximal and a second or distal side wall (1,2) of flexible sheet material sealed to each other for defining a cavity therebetween for receiving human stomal discharge through an aperture (8a) in said proximal side wall (1) and an elongate drainage portion extending downwards in a longitudinal direction of said pouch, said drainage portion ending in a drainage outlet (5) extending transversely to said longitudinal direction and formed along the bottom edges of said first and second side walls (1,2),
- a first or proximal and a second or distal flexible stiffening strip (6,7) located near said drainage outlet (5) and extending transversely to said longitudinal direction and being attached to the outer surfaces of said first and second side walls (1,2), respectively, of said drainage portion,
- a third, intermediate wall (3) of flexible sheet material arranged between said proximal and distal side walls (1,2) for dividing said cavity into two compartments, a proximal faeces compartment (3b) and a distal flatus gas compartment (3a), and ending at a transversely extending bottom edge (4) located in the vicinity of said drainage outlet (5), and
- securing means for securing a coil comprising said first and second stiffening strips (6,7) and said first, second and third walls (1,2,3), said coil being formed by rotating said strips (6,7) upwards at least 360 degrees, preferably at least 540 degrees,
**characterized in that**
said intermediate wall (3) is attached at one or more spot welds or adhesive spots to said distal wall (2) in the vicinity of said bottom edge (4) of said intermediate wall (3) such that if said stiffening strips (6,7) are arcuately flexed away from each other to open said drainage outlet (5) said intermediate wall (3) will follow said distal wall (2) and not interfere with the cleaning of said drainage outlet (5) after said pouch is emptied through said drainage outlet (5).

2. A pouch according to claim 1, wherein said bottom edges (4) of said first and second walls (1,2) and said intermediate wall (3) generally coincide.

3. A pouch according to claim 1 or 2, wherein said intermediate wall (3) is attached to said distal wall (2) by means of welding.

4. A pouch according to claim 3, wherein said welding is preferably in the form of four mutually spaced spot welds such that preferably two or more channels for draining any stomal effluent in said flatus gas compartment (3a) and for the escape of flatus gas that may be retained therein are formed between said spot welds (3c).

5. A pouch according to any of the preceding claims, wherein said stiffening strips (6,7) are generally rectangular.

6. A pouch according to any of the preceding claims, wherein said stiffening strips (6,7) generally overlie each other in generally parallel side-by-side relation.

7. A pouch according to any of the claims 5-6, wherein the bottom edges (4) of said stiffening strips (6,7) are located at a distance from said drainage outlet (5) that is less than 10 mm, preferably less than 5mm, most preferably less than 2mm.

8. A pouch according to any of the preceding claims, wherein said intermediate wall (3) is attached to said distal wall (2) at a distance of from said drainage outlet (5) that is less than 15mm, preferably less than 10mm, most preferably less than 8mm.

9. A pouch according to any of the preceding claims, wherein one or more flatus gas venting openings (8b,8c), preferably a plurality of such openings (8b), are provided in said intermediate wall (3) and a flatus gas venting aperture (8c) is provided in said distal wall (2).

10. A pouch according to claim 9, wherein said flatus gas venting openings (8b) in said intermediate wall (3) have a maximum dimension of between 0.01 mm and 0 5mm

11. A pouch according to claim 5, wherein said strips (6,7) are offset in directions extending transversely to said longitudinal direction so that each strip (6,7) has one end portion extending beyond the corresponding end portion of the other of the offset strips.

## Patentansprüche

1. Entwässerbarer Ostomiebeutel, enthaltend:
- eine erste oder proximale und eine zweite oder distale Seitenwand (1, 2) aus einem flexiblen Folienmaterial, die miteinander dichtend verbunden sind, um einen Hohlraum zwischen sich auszubilden, um menschliche Ausscheidungen eines künstlichen Darmausgangs durch eine Öffnung (8a) in der proximalen Seitenwand (1) und einen länglichen Drainageabschnitt aufzunehmen, der sich in Längsrichtung des Beutels nach unten erstreckt, wobei der Drainageabschnitt in einer Drainageöffnung (5) endet, die sich in einer Querrichtung im Bezug auf die Längsrichtung erstreckt und entlang der unteren Ränder der ersten und der zweiten Seitenwand (1, 2) ausgebildet ist,
- einen ersten oder proximalen und einen zweiten oder distalen, flexiblen Aussteifungsstreifen (6, 7), die sich in der Nähe der Drainageöffnung (5) befinden, quer zu der Längsrichtung verlaufen und an den Außenoberflächen der ersten bzw. zweiten Seitenwand (1, 2) des Drainageabschnittes angebracht sind,
- eine dritte, Zwischenwand (3) eines flexiblen Folienmaterials, die zwischen der proximalen und der distalen Seitenwand (1, 2) angeordnet ist, um den Hohlraum in zwei Abteile, ein proximales Fäkalabteil (3b) und ein distales Flatusgasabteil (3a), zu teilen, und an einem sich in Querrichtung erstreckenden unteren Rand (4) endet, der sich in der Nähe der Drainageöffnung (5) befindet, und
- eine Sicherungseinrichtung zum Sichern einer Wicklung, die den ersten und den zweiten Versteifungsstreifen (6, 7) sowie die erste, die zweite und die dritte Wand (1, 2, 3) enthält, wobei die Wicklung durch Drehen der streifen (6, 7) nach oben um wenigstens 360 Grad, vorzugsweise jedoch um 540 Grad ausgebildet wird,
**dadurch gekennzeichnet, dass**
die Zwischenwand (3) an wenigstens einem Schweißpunkt oder Klebepunkt an der distalen Wand (2) in der Nähe des unteren Randes (4) der Zwischenwand (3) derart angebracht ist, dass, wenn die Verstelfungsstreifen (6, 7) ordnungsgemäß voneinander weg gebogen werden, um die Drainageöffnung (5) zu öffnen, die Zwischenwand (3) der distalen Wand (2) folgt und bei der Reinigung der Drainageöfinung (5) nicht hinderlich ist, nachdem der Beutel durch die Drainageöfinung (5) geleert wurde.

2. Beutel nach Anspruch 1, bei dem die unteren Ränder (4) der ersten und der zweiten Wand (1, 2) und der Zwischenwand im wesentlichen übereinstimmen.

3. Beutel nach Anspruch 1 oder 2, bei dem die Zwischenwand (3) an der distalen Wand (2) durch Verschweißen angebracht ist.

4. Beutel nach Anspruch 3, bei dem die Verschweißung vorzugsweise die Gestalt vier zueinander beabstandeter Schweißpunkte hat, so dass vorzugsweise wenigstens zwei Kanäle zum Abführen von Ausfluss aus dem künstlichen Darmausgang in dem Flatusgasabteil (3a) und für das Entweichen von Flatusgas, das sich darin sammeln kann, zwischen den Schweißpunkten ausgebildet sind.

5. Beutel nach einem der vorhergehenden Ansprüche, bei dem die Versteifungsstreifen (6, 7) im wesentlichen rechteckig sind.

6. Beutel nach einem der vorhergehenden Ansprüche, bei dem sich die Versteifungssteifen (6, 7) im wesentlichen parallel nebeneinander überlappen,

7. Beutel nach einem der Ansprüche 5 bis 6, bei dem die unteren Ränder (4) der Versteifungsstreifen (6, 7) in einem Abstand von der Drainageöffnung (5) angeordnet sind, der geringer als 10 mm, vorzugsweise geringer als 5 mm und bestenfalls geringer als 2 mm ist.

8. Beutel nach einem der vorhergehenden Ansprüche, bei dem die Zwischenwand (3) an der distalen Wand (2) in einem Abstand von der Drainageöffnung (5) angeordnet ist, der geringer als 15 mm, vorzugsweise geringer als 10 mm und bestenfalls geringer als 8 mm ist.

9. Beutel nach einem der vorhergehenden Ansprüche, bei dem wenigstens eine Flatusgas-Entlüftungsöffnung (8b, 8c), vorzugsweise jedoch eine Vielzahl derartiger Öffnungen (8b) in der Zwischenwand (3) und eine Flatusgas-Entlüftungsöffnung (8c) in der distalen Wand (2) vorgesehen sind.

10. Beutel nach Anspruch 9, bei dem die Flatusgas-Entlüftungsöffnungen (8b) in der zwischenwand (3) eine maximale Abmessung zwischen 0,01 mm und 0,5 mm haben.

11. Beutel nach Anspruch 5, bei dem die Streifen (6, 7) in Richtungen, die sich quer zu der Längsrichtung erstrecken, derart versetzt sind, dass jeder Streifen (6, 7) einen Endabschnitt hat, der sich über den entsprechenden Endabschnitt des der versetzten Streifen hinaus erstreckt.

## Revendications

1. Poche de stomie vidangeable comprenant :
- une première ou proximale et une deuxième ou distale parois latérales (1, 2) d'une feuille de matériau flexible scellées l'une à l'autre afin de définir une cavité entre elles destinée à recevoir des écoulements humains de stomie via une ouverture (8a) dans ladite paroi latérale proximale (1) et une portion de vidange allongée s'étendant vers le bas dans une direction longitudinale de ladite poche, ladite portion de vidange se terminant dans une sortie de vidange (5) s'étendant transversalement vers ladite direction longitudinale et formée le long des bords inférieurs desdites première et deuxième parois latérales (1, 2),
- une première ou proximale et une seconde ou distale bandes de raidissement flexible (6, 7) situées près de ladite sortie de vidange (5) et s'étendant transversalement vers ladite direction longitudinale et étant fixées aux surfaces externes desdites première et seconde parties latérales (1, 2), respectivement, de ladite portion de vidange,
- une troisième paroi intermédiaire (3) en feuille de matériau flexible agencée entre lesdites parois latérales proximale et distale (1, 2) permettant de diviser ladite cavité en deux compartiments, un compartiment proximal pour les excréments (3b) et un compartiment distal pour les gaz de flatulence (3a), et se terminant à un bord inférieur s'étendant transversalement (4) situé à proximité de ladite sortie de vidange (5), et
- un moyen de fixation permettant de fixer une bobine comprenant lesdites première et seconde bandes de raidissement (6, 7) et lesdites première, deuxième et troisième parois (1, 2, 3), ladite bobine étant formée en pivotant lesdites bandes (6, 7) vers le haut d'au moins 360 degrés, de préférence d'au moins 540 degrés,
**caractérisée en ce que**
ladite paroi intermédiaire (3) est fixée à une ou plusieurs soudures par point ou un ou plusieurs points d'adhésif à ladite paroi distale (2) à proximité dudit bord inférieur (4) de ladite paroi intermédiaire (3) de sorte que si lesdites bandes de raidissement (6, 7) sont fléchies en position arquée à distance l'une de l'autre afin d'ouvrir ladite sortie de vidange (5), ladite paroi intermédiaire (3) suivra ladite paroi distale (2) et ne gênera pas le nettoyage de ladite sortie de vidange (5) après que ladite poche a été vidée via ladite sortie de vidange (5).

2. Poche selon la revendication 1, dans laquelle lesdits bords inférieurs (4) desdites première et deuxième parois (1, 2) et de ladite paroi intermédiaire (3) coïncident généralement.

3. Poche selon la revendication 1 ou 2, dans laquelle ladite paroi intermédiaire (3) est fixée à ladite paroi distale (2) au moyen d'un soudage.

4. Poche selon la revendication 3, dans laquelle ladite soudure est de préférence sous la forme de quatre soudures par point mutuellement espacées de sorte que de préférence deux ou plusieurs canaux permettant de vidanger tout effluent de la stomie dans ledit compartiment pour gaz de flatulence (3a) et permettant l'échappement des gaz de flatulence qui pourraient y être retenus sont formés entre lesdites soudures par point (3c).

5. Poche selon l'une quelconque des revendications précédentes, dans laquelle lesdites bandes de raidissement (6, 7) sont généralement rectangulaires.

6. Poche selon l'une quelconque des revendications précédentes, dans laquelle lesdites bandes de raidissement (6, 7) se recouvrent généralement l'une l'autre dans une relation généralement parallèle les unes à côté des autres.

7. Poche selon l'une quelconque des revendications 5 à 6, dans laquelle les bords inférieurs (4) desdites bandes de raidissement (6, 7) sont situés à une distance de ladite sortie de vidange (5) qui est inférieure à 10 mm, de préférence inférieure à 5 mm et de manière davantage préférée inférieure à 2 mm.

8. Poche selon l'une quelconque des revendications précédentes, dans laquelle ladite paroi intermédiaire (3) est fixée à ladite paroi distale (2) à une distance de ladite sortie de vidange (5) qui est inférieure à 15 mm, de préférence inférieure à 10 mm et de manière préférée entre toutes inférieure à 8 mm.

9. Poche selon l'une quelconque des revendications précédentes, dans laquelle une ou plusieurs ouvertures d'évacuation des gaz de flatulence (8b, 8c), de préférence une pluralité de telles ouvertures (8b) sont disposées dans ladite paroi intermédiaire (3) et une ouverture d'évacuation des gaz de flatulence (8c) est disposée dans ladite paroi distale (2).

10. Poche selon la revendication 9, dans laquelle lesdites ouvertures d'évacuation des gaz de flatulence (8b) dans ladite paroi intermédiaire (3) ont une dimension maximale comprise entre 0,01 mm et 0,5 mm.

11. Poche selon la revendication 5, dans laquelle lesdites bandes (6, 7) sont décalées dans des directions s'étendant transversalement vers ladite direction longitudinale de sorte que chaque bande (6, 7) a une portion d'extrémité s'étendant au-delà de la portion d'extrémité correspondante de l'autre des bandes décalées.
